# EUROPÄISCHE PATENTANMELDUNG

(11) **EP 1 862 468 A1**
(43) Veröffentlichungstag der Anmeldung: **05.12.2007**
(21) Anmeldenummer: 06090095.8
(22) Anmeldetag: 02.06.2006
(51) Int. Cl.: C07J 1/00, A61K 31/567, A61P 5/36

(54) **Kristallines 11beta-(4-Acetylphenyl)-20,20,21,21,21-pentafluor-17-hydroxy-19-nor-17alpha-pregna-4,9-dien-3-on**

(71) Anmelder: Bayer Schering Pharma Aktiengesellschaft, 13353 Berlin (DE)
(72) Erfinder: Beckmann, Wolfgang. Dr., 13465 Berlin (DE); Winter, Gabriele. Dr., 16567 Schönfliess (DE); Krämer, Edda. Dr, 14195 Berlin (DE); Ginko, Thomas, 13595 Berlin (DE); Amoulong, Evelin. Dr, 14612 Falkensee (DE)

(57) **Zusammenfassung**

Die vorliegende Erfindung betrifft kristallines 11β-(4-Acetylphenyl)-20,20,21,21,21-pentafluor-17-hydroxy-19-nor-17a-pregna-4,9-dien-3-on. Insbesondere betrifft die vorliegende Erfindung das kristalline Ansolvat von 11β-(4-Acetylphenyl)-20,20,21,21,21-pentafluor-17-hydroxy-19-nor-17α-pregna-4,9-dien-3-on, aber auch dessen Vorstufen, insbesondere die kristallinen Methanol- und Ethanolsolvate. Es werden Verfahren zur Herstellung des Ansolvat mittels Verdrängungskristallisation oder mittels Ausrühren beschrieben. Das erfindungsgemäße Ansolvat ist besonders geeignet zur Herstellung von Arzneimitteln.

## Beschreibung

Die vorliegende Erfindung betrifft kristallines 11β-(4-Acetylphenyl)-20,20,21,21,21-pentafluor-17-hydroxy-19-nor-17α-pregna-4,9-dien-3-on. Insbesondere betrifft die vorliegende Erfindung das kristalline Ansolvat von 11β-(4-Acetylphenyl)-20,20,21,21,21-pentafluor-17-hydroxy-19-nor-17α-pregna-4,9-dien-3-on, aber auch dessen Vorstufen, insbesondere die kristallinen Methanol- und Ethanolsolvate.

Für die Verarbeitung pharmazeutischer Wirkstoffe in oralen Arzneiformen müssen diese Wirkstoffe üblicherweise in fester Form vorliegen. Dabei sind eine Reihe von Festkörperformen möglich. Sie können amorph oder kristallin sein. Bei der Kristallisation ist es möglich, dass der Wirkstoff als Ansolvat anfällt. Ebenso ist es möglich, dass sich ein Solvat durch den Einbau von Lösemitteln in den Kristall bildet. Ein Hydrat ist beispielsweise ein Solvat, welches sich unter Einbau von Wasser in den Kristall gebildet hat.

Es ist bekannt, dass eine Reihe von physikalisch-chemischen Eigenschaftendurch die jeweilige Festkörperform bestimmt werden. Derartige Eigenschaften von pharmazeutischer Relevanz sind zum Beispiel die chemische Stabilität des Wirkstoffes, seine Stabilität gegenüber pharmazeutischen Hilfsstoffen, seine Vermahlbarkeit und sein Fließverhalten. Ebenfalls bekannt ist, dass kristalline Festkörper eine gegenüber amorphen Festkörpern höhere Stabilität aufweisen. Bei amorphen Festkörpern besteht die Gefahr der Rekristallisation und damit die Gefahr eines unkontrollierten Verlustes von der in der pharmazeutischen Formulierung eingesetzten Festkörperform. Der Vorteil amorpher Festkörper liegt unter anderem in dessen höherer Löslichkeit beziehungsweise deren deutlich erhöhten Lösegeschwindigkeit (Dissolution). Bei Auswahl der in einer konkreten pharmazeutischen Formulierung zu verwendenden Festkörperform eines Wirkstoffes sind Vorteile und Nachteile zum Beispiel bei der Lösegeschwindigkeit, der Stabilität und der Verarbeitbarkeit gegeneinander abzuwiegen. Eine stabile Festkörperform ist Voraussetzung für die Entwicklung eines Arzneimittels, da mit der Umwandlung von einer Festkörperform in eine andere immer auch Eigenschaftsänderungen verbunden sind.

Als kristalline Festkörper sind für pharmazeutische Anwendungen Ansolvate und Hydrate akzeptabel. Solvate nichtwässriger Lösungsmittel sind aufgrund des hohen organischen Lösemittelanteils - von wenigen Ausnahmen abgesehen - als Wirkstoff ungeeignet.

Die Herstellung fester pharmazeutischer Wirkstoffe umfasst unter anderem die chemische Synthese, die Aufreinigung und die Feststoffgewinnung. Für die Aufreinigung wird zunehmend die präparative Chromatographie eingesetzt. Sie ist in der Lage, Verunreinigungen in einen hohen Maße abzureichern, ohne dass es zu einem nennenswerten Verlust an Wirkstoff kommt. Das ist insbesondere für mit dem Wirkstoff chemisch eng verwandte Verunreinigungen von Vorteil, die bei der klassischen Kristallisation nur schlecht oder unter hohen Verlusten an Wirkstoff in der Mutterlauge abzureichern sind. Der Wirkstoff liegt im Raffinat der präparativen Chromatographiestufe relativ verdünnt vor. Aus diesem Raffinat muss der Wirkstoff in fester Form isoliert werden.

11 β-(4-Acetylphenyl)-20,20,21,21,21-pentafluor-17-hydroxy-19-nor-17α-pregna-4,9-dien-3-on hat die Strukturformel:

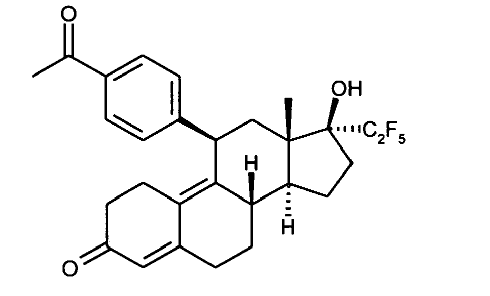

11 β-(4-Acetylphenyl)-20,20,21,21,21-pentafluor-17-hydroxy-19-nor-17α-pregna-4,9-dien-3-on ist bislang nur als amorpher Schaum bekannt (EP 0970103 B1, Seite 9. Absatz 0056). Dieser amorphe Schaum entsteht durch Einengen zur Trockne der den Wirkstoff enthaltenen Fraktionen nach der Chromatographie. Die so gewonnenen amorphen Schäume genügen bezüglich des Gehalts an Restlösemitteln den Anforderungen an einen pharmazeutischen Wirkstoff nicht. Außerdem ist die Abtrennung des Schaums aus dem Rührwerk schwierig. Ein weiterer Schritt auf dem Weg zur fertigen Formulierung ist die Mikronisierung. Mikronisierung ist dabei eine feine Vermahlung des Mahlgutes zum Beispiel mit einer Luftstrahlmühle. In Frage kommen aber auch alternative Herstellungsverfahren von Mikropartikeln. Diese ist insbesondere bei niedrigdosierten pharmazeutischen Zubereitungen nötig, um einen gleichmäßigen Gehalt an Wirkstoff in der Formulierung zu gewährleisten. Voraussetzung für eine gute Vermahlbarkeit einer Substanz ist unter anderem eine ausreichende Fließfähigkeit sowohl der Ausgangsware als auch des Mahlgutes. Auch hier ist die Handhabung der bislang bekannten Form schwierig, da sich diese elektrostatisch auflädt und daher nur schwer mikronisierbar ist.

Der übliche Weg zur Generierung eines handhabbaren Festkörpers durch Kristallisation aus Lösungen konnte bislang nicht gegangen werden. 11β-(4-Acetylphenyl)-20,20,21,21,21-pentafluor-17-hydroxy-19-nor-17a-pregna-4,9-dien-3-on bildet bei der Kristallisation aus den für die Kristallisation von Endstufen akzeptablen und gängigen Lösemitteln, in denen es sich ausreichend löst, Solvate. Diese wurden nach der Kristallisation aus organischen Lösemitteln wie zum Beispiel Methanol, Ethanol, Isopropanol, Aceton, 2-Butanon, Diisopropylether, Dioxan oder Tetrahydrofuran beziehungsweise aus den Lösemittelgemischen Isopropanol/Wasser, Ethanol/Essigester, Isopropanol/Isopropylacetat nachgewiesen. Diese Solvate erfüllen wegen ihres Gehalts an Restlösemittel jedoch die Anforderungen an einen pharmazeutischen Wirkstoff nicht. Die Entfernung des Lösemittels aus den so gebildeten Solvaten durch Trocknung führt wiederum zu einer amorphen Phase.

Es ist allgemein bekannt, dass das Auftreten von neuen, bislang nicht bekannten Festkörperformen einer bekannten chemischen Verbindung nicht vorhersagbar ist. Die Existenz kristalliner Phasen kann genau so wenig vorhergesagt werden, wie die Anzahl polymorpher Formen. Ebenso wenig können Bildungsbedingungen und Eigenschaften der einzelnen Formen prognostiziert werden.

Aufgabe der vorliegenden Erfindung ist es eine Festkörperform von 11 β-(4-Acetylphenyl)-20,20,21,21,21-pentafluor-17-hydroxy-19-nor-17a-pregna-4,9-dien-3-on zu generieren, die weder die Nachteile der bekannten amorphen Form, insbesondere die geringe Lagerstabilität und elektrostatische Aufladung bei der Verarbeitung, noch die Nachteile kristalliner Solvate mit organischen Lösungsmitteln aufweist.

Die Aufgabe wurde durch das Auffinden des kristallinen Ansolvates von 11β-(4-Acetylphenyl)-20,20,21,21,21-pentafluor-17-hydroxy-19-nor-17α-pregna-4,9-dien-3-on gelöst.

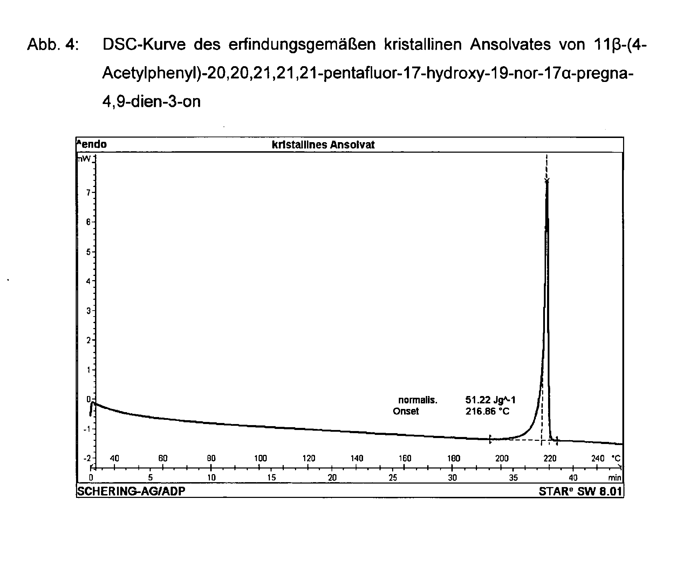

Es ist bekannt, dass amorphe Festkörperformen keinen gut definierten und aussagekräftigen Schmelzpunkt aufweisen. Die DSC-Kurve (DSC = Differential Scanning Calorimetrie) des aus EP 0970103 B1 bekannten amorphen Schaums zeigte abhängig von der gewählten Heizrate eine Exothermie zwischen 110°C und 200°C, gefolgt von einer Endothermie bei etwa 218°C (vergleiche Abbildung 1). Der nach der aufgetretenen Exothermie vorliegende Festkörper wurde mittels XRPD (XRPD = X-Ray Powder Diffraction; Röntgenpulverdiffraktometrie) untersucht. Eine neue, vollständig kristalline Form, die sich der Identifizierung in einem klassischen, aber auch in einem HTS-Screen (HTS = High Throughput Screen) durch Bildung von Solvaten entzieht, konnte so aufgefunden werden. Abbildung 2 zeigt das Röntgenpulverdiffraktogramm des amorphen Schaums, welches keine definierten XRPD-Linien zeigt. In Abbildung 3 ist das Röntgenpulverdiffraktogramm des erfindungsgemäßen kristallinen Ansolvat dargestellt (Transmission, Cu Kₐ₁-Strahlung, 20 - 25°C). Das kristalline Ansolvat zeigt eine XRPD-Linie d = 21,4 Å. Weitere XRPD-Linien liegen bei 5,3 Å, 7,7 Å und 5,8 Å. In Abbildung 4 ist die DSC-Kurve des kristallinen Ansolvates dargestellt, welches bei circa 218°C schmilzt. Das Infrarotspektrum (singlebounce ATR-IR) des erhaltenen Ansolvates zeigt Banden bei 1680 cm⁻¹, 1628 cm⁻¹ und 1215 cm⁻¹ (siehe Abbildung 5).

Es gelang die so gefundene kristalline Form auch in größerem Maßstab (kg-Bereich) herzustellen. Die hierbei angewandten Verfahren sind die Verdrängungskristallisation mittels Wasser und das Ausrühren .

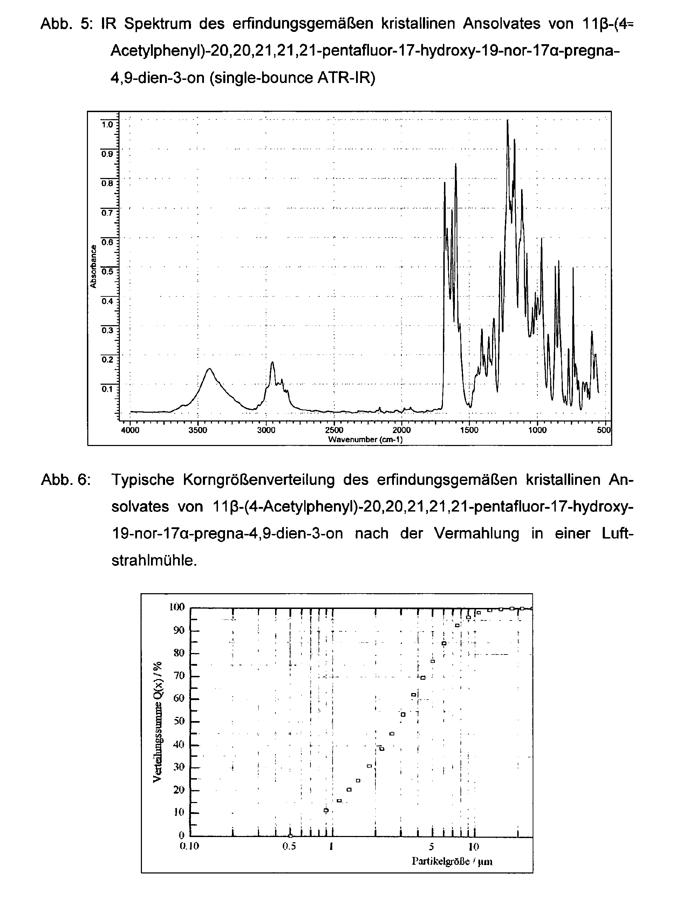

Das erfindungsgemäße kristalline Ansolvat von 11β-(4-Acetylphenyl)-20,20,21,21,21 -pentaftuor-17-hydroxy-19-nor-17α-pregna-4,9-dien-3-on weist neben den vorn genannten Vorteilen eine Reihe von weiteren Eigenschaften auf, die sich positiv auf die pharmazeutische Verarbeitung auswirken. Es lädt sich nicht elektrostatisch auf und lässt sich daher problemlos in der Luftstrahlmühle mikronisieren. Abbildung 6 zeigt eine typische Verteilungskurve des Mahlgutes. Eine Korngrößensummenverteilung bei der mehr als 50 % aller Partikel einen Durchmesser von kleiner/gleich 3 µm (für die untere Verteilung, gemessen an der volumenbezogenen Partikelgrößenverteilung) besitzen (so genannter x_{50,3}-Wert) lässt sich für das amorphe Material nur sehr schlecht und insbesondere nicht in einem industriellen Maßstab erzielen, da die elektrostatische Aufladung und die damit verbundene schlechte Fließfähigkeit eine gezielte Dosierung in die Mühle extrem erschwert.

Bei der Mikronisierung des erfindungsgemäßen kristallinen Ansolvates sinkt der Anteil an Restlösemittel weiter. Die entsprechenden Werte können Tabelle 1 entnommen werden. Der Restlösemittelgehalt des erfindungsgemäßen kristallinen Ansolvates liegt nach der Mikronisierung mit 0,34 - 0,35 % unterhalb des in der ICH-Q3C-Richtlinie (CPMP/ICH/283/95, 4.3, Seite 8/18) für Ethanol empfohlenen Wertes von 0,5 %. Laut Röntgenpulverdiffraktogramm liegt vor und nach der Mikronisierung das kristalline Ansolvat ohne jegliche Anteile von Ethanolsolvat vor.

**Tab. 1: Ethanolanteil im erfindungsgemäßen kristallinen Ansolvat vor und nach der Vermahlung in einer Luftstrahlmühle (Mikronisierung)**

| | Ethanolanteil | |
|---|---|---|
| Charge | vor der Vermahlung | nach der Vermahlung |
| I | 1,08 % | 0,35 % |
| II | 1,00 % | 0,34 % |
| III | 1,24 % | 0,35 % |

Das kristalline Ansolvat weist gegenüber der amorphen Form eine überragende Stabilität auf. Dies zeigt sich beim Vergleich der Ergebnisse der Temperatur-, Feuchtigkeits- und insbesondere bei Lichtbelastungstests. In Tabelle 2 ist die Abnahme des Wirkstoffgehalts unter Lagerung bei erhöhter Temperatur und erhöhter Feuchte aufgeführt. Vor Lagerung wies das eingesetzte Material einen Gehalt von 98,4 % beziehungsweise von 95,4 % auf.

**Tab. 2: Vergleich der Kurzzeitstabilität von amorphem 11 β-(4-Acetylphenyl)-20,20,21,21,21-pentafluor-17-hydroxy-19-nor-17α-pregna-4,9-dien-3-on und dem erfindungsgemäßen kristallinen Ansolvat bei Lagerung unter erhöhter Temperatur und erhöhter Luftfeuchte. Angegeben ist die Abnahme des Wirkstoffgehaltes.**

| Temperatur | rel. Luftfeuchte | **amorph** | | **kristallin** | |
|---|---|---|---|---|---|
| ϑ | ϕ | 15 Tage | 30 Tage | 15 Tage | 30 Tage |
| 50°C | | - 2,9 % | -5.1 % | 0 % | - 0,3 % |
| 50°C | 75 % | - 2,8 % | - 3,3 % | 0 % | - 0,1 % |
| 70°C | | - 10,2 % | - 17,3 % | - 1,5 % | - 3,5 % |
| 70°C | 75 % | - 13,5 % | - 17,4 % | - 0,1 % | - 0,2 % |
| 90°C | | - 32,6 % | k. A. | - 3,6 % | k. A. |
| 90°C | 75 % | - 31,7 % | k. A. | - 0,2 % | k. A. |

Noch deutlicher wird die höhere Stabilität des erfindungsgemäßen kristallinen Ansolvats bei Lagerung unter Licht. In Tabelle 3 sind die Stabilitäten nach Lagerung unter 20 kLux für 42 Stunden und für 66 Stunden aufgeführt. Die Startwerte lagen auch hier bei 98,4 % beziehungsweise 95,4 %.

**Tab. 3: Vergleich der Stabilität von amorphem 11β-(4-Acetylphenyl)-20,20,21,21,21-pentafluor-17-hydroxy-19-nor-17α-pregna-4,9-dien-3-on und dem erfindungsgemäßen kristallinen Ansolvat bei Lagerung unter Licht. Angegeben ist die Abnahme des Wirkstoffgehaltes.**

| Dauer | **amorph** | **kristallin** |
|---|---|---|
| 42 h | - 34 % | - 0,2 % |
| 66 h | -42 % | -0,4 % |

Beim Einsatz von 11β-(4-Acetylphenyl)-20,20,21,21,21-pentafluor-17-hydroxy-19-nor-17a-pregna-4,9-dien-3-on als Wirkstoff in pharmazeutischen Zubereitungen ist das Verunreinigungsprofil von entscheidender Bedeutung. Eine bei der Lagerung dieses Wirkstoffes auftretende Verbindung ist 11β-(4-Acetylphenyl)-20,20,21,21,21-pentafluor-17-hydroxy-9,10-epoxy-19-nor-1 Oa,17a-pregna-1,4-dien-3-on. Die Toxizität dieser Verbindung ist bekannt. Der Gehalt dieser Verunreinigung muss bis zum Ablauf der Haltbarkeitsdauer der pharmazeutischen Formulierung unter 0,2 % liegen. Für den amorphen Festkörper von 11β-(4-Acetylphenyl)-20,20,21,21,21-pentaftuor-17-hydroxy-l 9-nor-17a-pregna-4,9-dien-3-on wurde bei Lagerung unter Stressbedingungen (erhöhte Temperatur und Luftfeuchte) und unter Licht eine erhebliche Bildung dieser Verunreinigung gefunden. Der amorphe Festkörper eignet sich daher ohne Stabilisierung nicht zum Einsatz in einem Arzneimittel. Beim kristallinen Festkörper indes ist die Zunahme dieser kritischen Verunreinigung nahezu Null. Eine aufwendige Stabilisierung bei Verwendung des kristallinen Ansolvats ist daher nicht mehr erforderlich. Bei der Lagerung von amorphem 11 β-(4-Acetylphenyl)-20,20,21,21,21-pentafluor-17-hydroxy-19-nor-17α-pregna-4,9-dien-3-on bei einer Temperatur von 70°C entstehen bereits nach 15 Tagen 0,6 %, nach 30 Tagen schon 1,1 % der oben genannten Epoxyverunreinigung. Lagert man hingegen das erfindungsgemäße kristalline Ansolvat bei der selben Temperatur 30 Tage so sind gerade 0,1 % der Epoxyverunreinigung nachweisbar. Die Tabelle 4 zeigt die Zunahme der Epoxy-Verunreinigung bei der Lagerung von amorphen 11β-(4-Acetylphenyl)-20,20,21,21,21 -pentafluor-1 7-hydroxy-1 9-nor-1 7α-pregna-4,9-dien-3-on unter Stressbedingungen beziehungsweise unter Licht. Im Vergleich dazu weist das erfindungsgemäße kristalline Ansolvat eine Zunahme dieser Verunreinigungen von unter 0,2 % auf.

**Tab. 4: Zunahme der Epoxy-Verunreinigung bei Lagerung von amorphem 11 β-(4-Acetylphenyl)-20,20,21,21,21-pentafluor-17-hydroxy-19-nor-17α-pregna-4,9-dien-3-on und dem erfindungsgemäßen kristallinen Ansolvat bei Lagerung unter Licht.**

| Bedingungen | | **amorph** | **kristallin** |
|---|---|---|---|
| 20 kLux | 42 h | 8,4 % | 0,1 % |
| 20 kLux | 66 h | 11,2 % | 0,1 % |

Für den amorphen Wirkstoff wurde unter Stressbedingungen (15 d, 90°C/75 % relative Luftfeuchtigkeit) eine teilweise Rekristallisation gefunden. Es ist davon auszugehen, dass eine solche Rekristallisation auch auftritt, wenn die amorphe Phase über einen längeren Zeitraum bei niedrigeren Temperaturen gelagert wird. Eine solche Umwandlung ist aber in der fertigen Arzneiform unerwünscht, da dies zu einem veränderten, nicht reproduzierbaren Freisetzung des Wirkstoffes führen, aber auch die Härte der Arzneiform beeinflusst sein kann.

Das erfindungsgemäße kristalline Ansolvat kann zu pharmazeutischen Zubereitungen verarbeitet werden, welche zur Behandlung von Myomen oder eines Mammacarcinoms eingesetzt werden können. Es kann als Wirkstoff bei der weiblichen Kontrazeption, aber auch zur Behandlung gynäkologischer Störungen, wie der Dysmenorrhoe oder der Endometriose, zur Hormonersatztherapie, zur Menstruationsauslösung und zur Geburtseinleitung verwendet werden. Aufgrund seiner starken Antitumor-Aktivität kann es auch in Kombination mit einem Antiestrogen (gleichzeitig oder sequentiell) in Präparaten zur Behandlung hormonabhängiger Tumoren (EP0310542) eingesetzt werden. Denkbar ist auch eine Verwendung bei der Behandlung von Tumoren im Darmbereich, im Bereich der Prostata, des Eierstocks, des Endometriums und von Meningomen.

11 β-(4-Acetylphenyl)-20,20,21,21,21-pentafluor-17-hydroxy-19-nor-17α-pregna-4,9-dien-3-on bildet mit den für diese Substanz in Frage kommenden Lösemitteln Solvate. Zur Herstellung des erfindungsgemäßen Ansolvates sind zwei Wege möglich: Zum einen kann das Ansolvat durch Verdrängung mittels Wasser, zum anderen mittels Ausrühren hergestellt werden.

Das erfindungsgemäße Ansolvat kann aus einem organischen Lösemittel durch eine Verdrängungskristallisation erhalten werden. Dabei darf 11β-(4-Acetylphenyl)-20,20,21,21,21-pentafluor-17-hydroxy-19-nor-17α-pregna-4,9-dien-3-on mit dem zur Verdrängung eingesetzten Anti-Solvent kein Solvat bilden. Als Primärlösemittel können auch solche eingesetzt werden, mit denen 11β-(4-Acetylphenyl)-20,20,21,21,21-pentafluor-17-hydroxy-19-nor-17α-pregna-4,9-dien-3-on ein Solvat bildet, dann muss jedoch der Anteil an primärem Lösemittel bei der Verdrängung soweit herabgesetzt werden, dass das Solvat instabil wird. Ein mögliches Anti-Solvent ist Wasser, da 11β-(4-Acetylphenyl)-20,20,21,21,21-pentafluor-17-hydroxy-19-nor-17a-pregna-4,9-dien-3-on keine Hydrate ausbildet. Der für die Vermeidung der Bildung des Solvats erforderliche Anteil Wasser hängt sowohl vom Primärlösemittel ab, als auch von der Temperatur bei der die Kristallisation geführt wird. In Tabelle 5 sind die für das Primärlösemittel Ethanol erforderlichen Anteile Wasser im Ethanol als Funktion der Temperatur aufgeführt, die für eine sichere Kristallisation des Ansolvates aus Ethanol mindestens erforderlich sind. Bei Raumtemperatur (20°C) sind zum Beispiel 40 wt-% Wasser erforderlich. 40 wt-% bedeutet dabei 40 Gewichtsprozent Wasser, also je Gramm Lösemittelmischung sind 0,4 g Wasser enthalten.

**Tab. 5: Zur sicheren Verdrängungskristallisation des erfindungsgemäßen kristallinen Ansolvates mindestens erforderlicher Wasseranteil in Abhängigkeit von der Temperatur**

| Temperatur | Wasseranteil |
|---|---|
| 0°C | 20 wt-% |
| 20°C | 40 wt-% |
| 60°C | 80 wt-% |

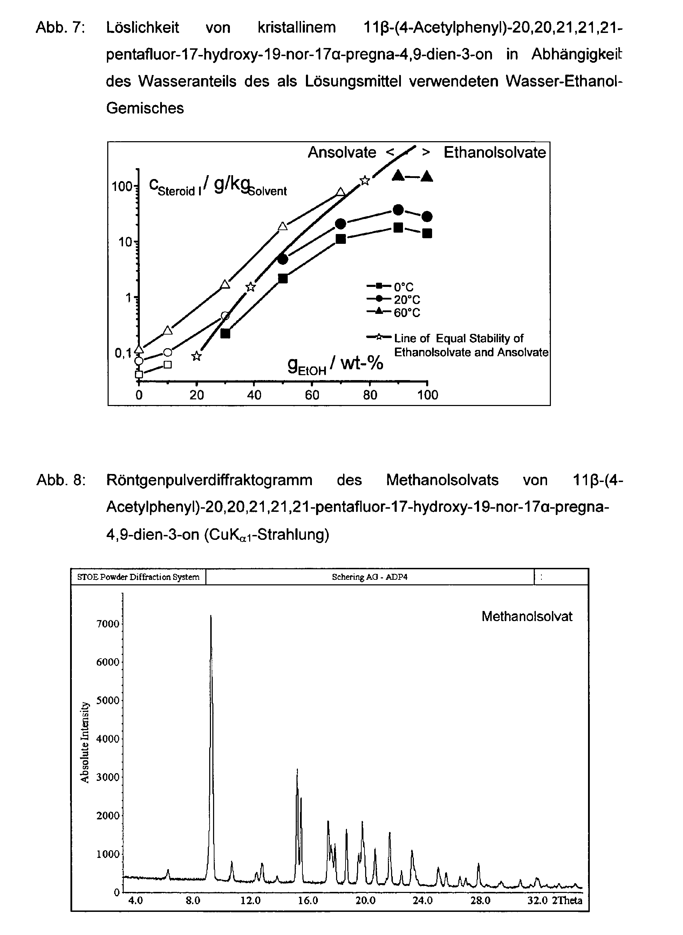

Die Löslichkeit von 11β-(4-Acetylphenyl)-20,20,21,21,21-pentafluor-17-hydroxy-19-nor-17α-pregna-4,9-dien-3-on in einem Wasser-Ethanol-Gemisch weist eine starke Abhängigkeit vom Wasseranteil auf. Diese Abhängigkeit ist in Abbildung 7 dargestellt. Die Löslichkeit von 11β-(4-Acetylphenyl)-20,20,21,21,21-pentafluor-17-hydroxy-19-nor-17α-pregna-4,9-dien-3-on ist bei einem Anteil von 20 wt-% Wasser bereits auf ein Hundertstel der Löslichkeit in reinem Ethanol gesunken ist. Damit ist für 11β-(4-Acetylphenyl)-20,20,21,21,21-pentafluor-17-hydroxy-19-nor-17α-pregna-4,9-dien-3-on die beschriebene Verdrängung wirtschaftlich sinnvoll. Für andere Systeme liegt der erforderliche Wasseranteil deutlich höher, so dass die Verdrängung nur sehr verdünnt und damit mit einer ungenügenden Raumausbeute durchgeführt werden kann.

Das erfindungsgemäße Ansolvat kann auch durch Ausrühren erhalten werden. Es ist bekannt, dass beim Ausrühren in einem Lösemittel mit geringer Lösekraft Phasenumwandlungen zwischen verschiedenen Festköperformen möglich sind. Die Umwandlung führt dabei immer zu dem unter den konkreten Bedingungen stabileren Festkörper. Bei Ausrühren von Solvaten kann es zur Entfernung des Lösemittels der Solvatisierung kommen. Hierzu muss die Stabilitätsdomäne für das Solvat verlassen werden. Wie oben beschrieben sind hierzu bei Raumtemperatur 40 wt-% Wasser in Ethanol ausreichend. Die Löslichkeit von 11β-(4-Acetylphenyl)-20,20,21,21,21-pentafluor-17-hydroxy-19-nor-17α-pregna-4,9-dien-3-on ist in einem solchen Gemisch bereits ausreichend niedrig, vergleiche Abbildung 7, so dass der Prozess ohne großen Subtanzverlust durchgeführt werden kann. Ob die bei einer derartigen Ausrührung entstehende neue Phase amorph oder kristallin ist, kann theoretisch nicht vorhergesagt werden. Erfindungsgemäß entsteht bei der beschriebenen Ausrührung ein kristallines Ansolvat.

### Beispiel 1: Rekristallisation unter thermischer Belastung

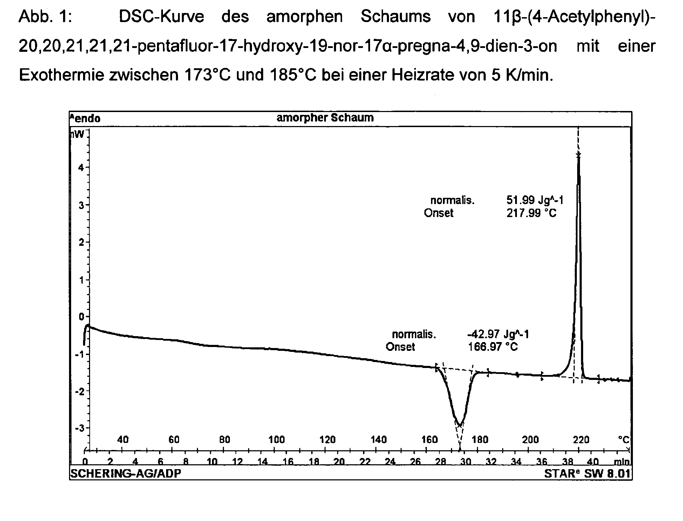

Zwischen 2 mg und 10 mg des amorphen Materials wurden in einer DSC mit Heizraten zwischen 1 K/min und 20 K/min in einer offenen Al-Kapsel unter Stickstoff erwärmt. Das Thermogramm zeigt eine Rekristallisationsexothermie, welche von einer Schmelzendothermie mit einer Onset-Temperatur von 218°C gefolgt wird (siehe Abbildung 1).

### Beispiel 2: Verdrängungskristallisation

Zu einer Lösung von 12,5 kg 11 β-(4-Acetylphenyl)-20,20,21,21,21-pentafluor-17-hydroxy-19-nor-17α-pregna-4,9-dien-3-on in 120 I Ethanol bei 60°C werden innerhalb von 10 Minuten 115 kg Wasser gegeben und bei einer Manteltemperatur von 60°C im Vakuum kodestilliert. Die Kodestillation wird so häufig wiederholt, bis der Ethanolgehalt im Dampf unter 1 % liegt. Anschließend wird auf 20°C gekühlt und noch 30 min nachgerührt. Nach Abtrennung des Feststoffes und Trocknung werden 11,9 kg kristallines Ansolvat erhalten.

### Beispiel 3: Verdrängungskristallisation unter Aufreinigung

Zu einer Lösung von 7,6 kg 11β-(4-Acetylphenyl)-20,20,21,21,21-pentafluor-17-hydroxy-19-nor-17α-pregna-4,9-dien-3-on in 33 I Ethanol werden bei Siedehitze innerhalb von 5 Minuten 58 kg Wasser gegeben. Anschließend wird auf 2°C abgekühlt und eine Stunde nachgerührt. Nach Abtrennung des Feststoffes und Trocknung werden 6,2 kg kristallines Ansolvat erhalten.

Bei der Verdrängung wurde bei einer Ausbeute von 93 % die Abreicherung bestimmter Verunreinigungen um einen Faktor von circa 3 erreicht. So nimmt 11β-(4-Acetylphenyl)-17β-hydroxy-17α-methylestra-4,9-dien-3-one von 1,1 % auf 0,38 % und damit unter die Spezifikation ab. Diese Verunreinigung ist anschließend in der Mutterlauge zu 63 % vorhanden.

### Beispiel 4: Ausrührung

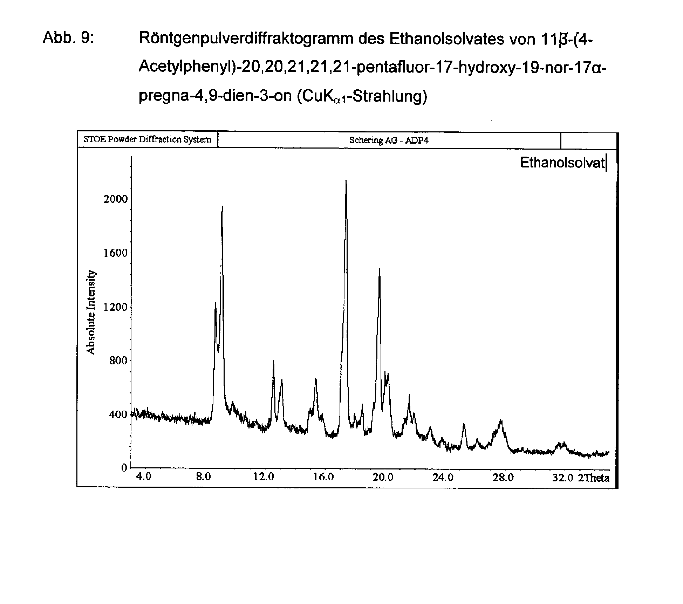

15,6 kg des Ethanolsolvats (Röntgenpulverdiffraktogramm: vergleiche Abbildung 9, Herstellung analog Beispiel 5) werden in 217 kg Wasser für eine Stunde bei einer Innentemperatur von 85°C ausgerührt. Anschließend wird auf 25°C gekühlt. Nach Isolierung und Trocknung werden 12,7 kg kristallines Ansolvat erhalten.

### Beispiel 5: Ausrührung

**585** mg von 11β-(4-Acetylphenyl)-20,20,21,21,21-pentafluor-17-hydroxy-19-nor-17a-pregna-4,9-dien-3-on werden in Methanol bei 64°C gelöst und durch Kühlen auf Raumtemperatur als Methanolsolvat erhalten. Nach Isolation und Trocknung werden 463 mg Methanolsolvat erhalten. Abbildung 8 zeigt das Röntgenpulverdiffraktogramm des Methanolsolvates.

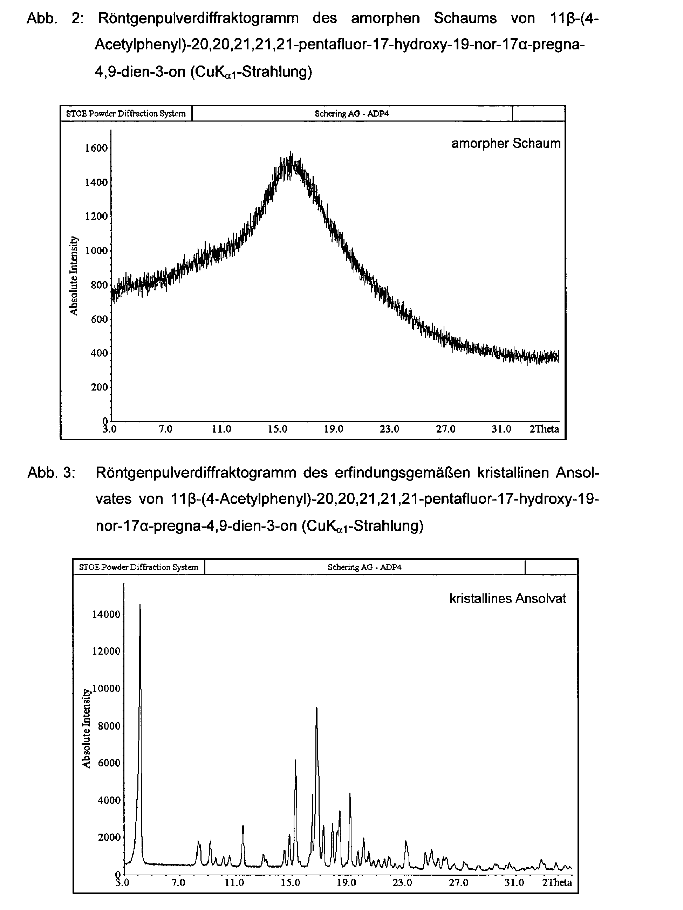

102 mg dieses Methanolsolvat werden in 5 mL Wasser bei 70°C für 245 min ausgerührt. Nach 31 min wird eine Probe entnommen und bei Raumtemperatur getrocknet. Das aufgenommene Röntgenpulverdiffraktogramm entspricht dem Röntgenpulverdiffraktogramm des Ansolvates (vergleiche Abbildung 3). Das Produkt enthält weniger als 0,02 % Methanol.

### Beispiel 6: Mikronisierung

10 kg des erfindungsgemäßen kristallinen Ansolvates, mit einem Restlösemittelgehalt von leicht oberhalb 1 % Ethanol (vergleiche Tabelle 1) werden mit einer Luftstrahlmühle bei einem Massenstrom von 4 kg/h und mit einem Mahldruck von 5 bar bei circa 220 Nm³/h vermahlen. Die gezielte Dosierung des Mahlgutes verläuft mangels elektrostatischer Aufladung problemlos. Das erhaltene Produkt weist Korngrößensummenverteilung (X_{50,3}-Wert) von 3 µm auf. Der Gehalt an Restlösemittel ist auf 0,35 % gesunken.

## Patentansprüche

1. Kristallines 11β-(4-Acetylphenyl)-20,20,21,21,21-pentafluor-17-hydroxy-19-nor-17α-pregna-4,9-dien-3-on.

2. Kristallines Ansolvat von 11β-(4-Acetylphenyl)-20,20,21,21,21-pentafluor-17-hydroxy-19-nor-17α-pregna-4,9-dien-3-on nach Anspruch 1.

3. Kristallines Solvat von 11β-(4-Acetylphenyl)-20,20,21,21,21-pentafluor-17-hydroxy-19-nor-17α-pregna-4,9-dien-3-on nach Anspruch 1.

4. Kristallines Methanolsolvat von 11β-(4-Acetylphenyl)-20,20,21,21,21-pentafluor-17-hydroxy-19-nor-17α-pregna-4,9-dien-3-on nach Anspruch 3.

5. Kristallines Ethanolsolvat von 11β-(4-Acetylphenyl)-20,20,21,21,21-pentafluor-17-hydroxy-19-nor-17α-pregna-4,9-dien-3-on nach Anspruch 3.

6. Kristallines Ansolvat nach Anspruch 2, **dadurch gekennzeichnet, dass** dessen Röntgen-Pulver-Diffraktogramm Linien bei d = 21,4 Å zeigt.

7. Kristallines Ansolvat nach Anspruch 2, **dadurch gekennzeichnet, dass** dessen Röntgen-Pulver-Diffraktogramm Linien bei d = 21,4 Å, d = 7,7 Å und d = 5,8 Å, d = 5,3 Å zeigt.

8. Kristallines Ansolvat nach Anspruch 2, **dadurch gekennzeichnet, dass** dessen IR-Spektrum Banden bei 1680 cm⁻¹, 1628 cm⁻¹ und 1215 cm⁻¹ zeigt.

9. Mikronisiertes kristallines Ansolvat von 11β-(4-Acetylphenyl)-20,20,21,21,21-pentafluor-17-hydroxy-19-nor-17a-pregna-4,9-dien-3-on nach einem der Ansprüche 2, 6, 7 oder 8.

10. Mikronisiertes kristallines Ansolvat von 11β-(4-Acetylphenyl)-20,20,21,21,21-pentafluor-17-hydroxy-19-nor-17α-pregna-4,9-dien-3-on nach Anspruch 9, **dadurch gekennzeichnet, dass** es einen Restlösemittelgehalt von unter 0,5 wt-% hat.

11. Mikronisiertes kristallines 11β-(4-Acetylphenyl)-20,20,21,21,21-pentafluor-17-hydroxy-19-nor-17a-pregna-4,9-dien-3-on nach Anspruch 9 oder 10, **dadurch gekennzeichnet, dass** dessen X_{50,3}-Wert (volumenbezogene Partikelgrößensummenverteilung) im Bereich von kleiner 5 µm und größer 1 µm liegt.

12. Pharmazeutische Zusammensetzung enthaltend ein kristallines 11β-(4-Acetylphenyl)-20,20,21,21,21-pentafluor-17-hydroxy-19-nor-17a-pregna-4,9-dien-3-on gemäß einem der Ansprüche 1 bis 11.

13. Pharmazeutische Zusammensetzung nach Anspruch 12 zur Behandlung von Myomen.

14. Pharmazeutische Zusammensetzung nach Anspruch 12 zur Behandlung von Brustkrebs.

15. Pharmazeutische Zusammensetzung enthaltend 11 β-(4-Acetylphenyl)-20,20,21,21,21-pentafluor-17-hydroxy-19-nor-17a-pregna-4,9-dien-3-on aber weniger als 0,2 % 11β-(4-Acetylphenyl)-20,20,21,21,21-pentafluor-17-hydroxy-9,10-epoxy-19-nor-10α,17a-pregna-1,4-dien-3-on.

16. Verfahren zur Herstellung des kristallinen Ansolvates gemäß Anspruch 2 durch Verdrängungskristallisation aus organischen Lösemitteln mit einem Antisolvent, mit dem 11β-(4-Acetylphenyl)-20,20,21,21,21-pentafluor-17-hydroxy-19-nor-17α-pregna-4,9-dien-3-on kein Solvat bildet.

17. Verfahren nach Anspruch 16, wobei das organische Lösungsmittel Ethanol und das Antisolvent Wasser ist.

18. Verfahren nach Anspruch 17, wobei der Wasseranteil oberhalb von 50 wt-% und die Temperatur unterhalb von 50°C liegt.

19. Verfahren zur Herstellung des kristallinen Ansolvates gemäß Anspruch 2 durch Ausrühren eines organischen Solvats in einem Lösemittel mit dem 11 β-(4-Acetylphenyl)-20,20,21,21,21-pentafluor-17-hydroxy-19-nor-17α-pregna-4,9-dien-3-on kein Solvat bildet.

20. Verfahren nach Anspruch 19, wobei das Solvat das Ethanolsolvat und das Lösungsmittel Wasser ist.

21. Verfahren nach Anspruch 20, wobei das Ausrühren bei einer Temperatur von 50 - 100°C durchgeführt wird.

22. Verfahren nach Anspruch 21, wobei das Ausrühren bei einer Temperatur von etwa 80 - 90°C durchgeführt wird.
